# EUROPÄISCHE PATENTSCHRIFT

(11) **EP 1 007 203 B1**
(45) Veröffentlichungstag und Bekanntmachung des Hinweises auf die Patenterteilung: **03.11.2004**
(21) Anmeldenummer: 98939655.1
(22) Anmeldetag: 05.08.1998
(51) Int. Cl.: B01J 23/56, B01J 23/656, C07C 68/00

(54) **VERFAHREN ZUR HERSTELLUNG VON DIARYLCARBONATEN MITTELS AUS EINEM SOL-GEL VERFAHREN HERGESTELLTEN PLATINMETALL ENTHALTENDEN KATALYSATOR**
METHOD FOR PRODUCING DIARYLCARBONATES USING CATALYSTS CONTAINING A PLATINUM GROUP METAL PRODUCED IN A SOL-GEL METHOD
PROCEDE POUR LA PREPARATION DE DIARYLCARBONATES EN PRESENCE DE CATALYSEURS CONTENANT UN METAL DU GROUPE DU PLATINE ET PREPARES A PARTIR D'UN PROCEDE SOL-GEL

(30) Priorität: 18.08.1997 DE 19735770
(43) Veröffentlichungstag der Anmeldung: 14.06.2000
(73) Patentinhaber: Bayer MaterialScience AG, 51368 Leverkusen (DE)
(72) Erfinder: HESSE, Carsten, D-47918 Tönisvorst (DE); NOTHEIS, Ulrich, D-41539 Dormagen (DE); RECHNER, Johann, D-47906 Kempen (DE)
(86) Internationale Anmeldenummer: PCT/EP1998/004862
(87) Internationale Veröffentlichungsnummer: WO 1999/008787

(56) Entgegenhaltungen:
- EP-A- 0 177 198
- EP-A- 0 602 865
- EP-A- 0 736 325
- EP-A- 0 745 426
- PATENT ABSTRACTS OF JAPAN vol. 010, no. 063 (C-332), 13. März 1986 & JP 60 202829 A (KOGYO GIJUTSUIN;OTHERS: 0J), 14. Oktober 1985

## Beschreibung

Die vorliegende Erfindung betrifft Platinmetall enthaltende Mischoxidkatalysatoren, die in einem Sol-Gel-Prozess hergestellt wurden, und ihren Einsatz in Verfahren zur Herstellung von Diarylcarbonaten durch Umsetzung von aromatischen Hydroxyverbindungen mit Kohlenmonoxid und Sauerstoff.

Es ist bekannt, organische Carbonate durch oxidative Umsetzung von aromatischen Hydroxyverbindungen mit Kohlenmonoxid in Gegenwart eines Edelmetall-Katalysators herzustellen (DE-OS 28 15 512). Als Edelmetall wird bevorzugt Palladium eingesetzt. Zusätzlich können ein Cokatalysator (z.B. Mangan- oder Kobaltsalze) eine Base, ein quaternäres Salz, verschiedene Chinone bzw. Hydrochinone und Trockenmittel eingesetzt werden. Dabei kann in einem Lösungsmittel, bevorzugt in Methylenchlorid, gearbeitet werden.

Für die wirtschaftliche Durchführung dieses Prozesses ist neben der Aktivität und der Selektivität die effektive Wiedergewinnung des Edelmetall-Katalysators von entscheidender Bedeutung: Zum einen stellt der Edelmetall-Katalysator einen erheblichen Kostenfaktor dar. Verluste an Edelmetall-Katalysator müssen kostenintensiv ersetzt werden. Zum anderen dürfen keine Reste des Edelmetall-Katalysators im Produkt verbleiben. Für den Prozeß der oxidativen Carbonylierung von aromatischen Hydroxyverbindungen zu Diarylcarbonaten ist die wirtschaftliche und effiziente Rückgewinnung homogener Katalysatoren bisher nicht beschrieben. Mit geringerem Aufwand kann die Abtrennung eines Edelmetall-Katalysators aus einer flüssigen Reaktionsmischung z.B durch Filtrieren oder Zentrifugieren erfolgen, wenn man heterogene Träger-Katalysatoren einsetzt.

In EP-A 572 980, EP-A 503 581 und EP-A 614 876 werden Edelmetall-Träger Katalysatoren verwendet, die 5% Palladium auf Kohleträgern enthalten. Jedoch liefern derartige Träger-Katalysatoren nur sehr unbefriedigende oder gar keine Umsätze, so daß auch diese für eine wirtschaftliche Prozeßführung nicht geeignet sind.

In JP-A 01/165 551 (zitiert nach C.A 112:76618j (1990)) wird beschrieben, daß für die Herstellung von aromatischen Carbonaten Palladium oder Pd-Verbindungen, wie Pdacetylacetonat, in Kombination mit (Erd)Alkaliiodiden oder Oniumiodiden, wie Tetrabutylammoniumiodid, und mindestens einem Zeolith eingesetzt werden können.

In JP-A 04/257 546 und JP-A 04/261 142 wird in je einem Beispiel ein Träger-Katalysator zur Herstellung von aromatischen Carbonaten beschrieben, bei dem Siliciumcarbid-Granulat als Trägermaterial für einen Träger-Katalysator in einer Destillationskolonne verwendet wird. Obwohl in den betreffenden Beispielen unter drastischen Bedingungen (hoher Druck, hohe Temperatur) gearbeitet wird, ermöglicht dieser Katalysator nur sehr geringe Raum-Zeit-Ausbeuten. Diese niedrigen Raum-Zeit-Ausbeuten machen eine ökonomische Herstellung von aromatischen Carbonaten mit derartigen Träger-Katalysatoren unmöglich.

EP-A 736 324 beschreibt die Herstellung von Diarylcarbonaten mit heterogenen Katalysatoren, die ein Platinmetall, bevorzugt Palladium, und eine cokatalytisch wirkende Metallverbindung, bevorzugt eines Metalles aus der Gruppe von Mn, Cu, Co, Ce und Mo enthalten. Bei der Herstellung der Katalysatoren werden die cokatalytisch wirkenden Metalle auf einen Träger aufgebracht.

EP-A 736 325 beschreibt die Herstellung von Diarylcarbonaten mit heterogenen Katalysatoren, die ein Platinmetall, bevorzugt Palladium, enthalten, auf einem Träger, der aus einem Metalloxid, dessen Metall in mehreren Wertigkeitsstufen auftreten kann, besteht.

Zwar ermöglichen diese Träger-Katalysatoren erstmals die Herstellung von aromatischen Carbonaten, dennoch ist aus ökonomischer Sicht eine weitere Steigerung der Aktivität wünschenswert.

Es wurde nun gefunden, daß sich höhere Katalysatoraktivitäten erhalten lassen, wenn die Katalysatoren in einem Sol-Gel-Prozess hergestellt wurden.

Gegenstand der Erfindung ist ein Verfahren zur Herstellung eines organischen Carbonats durch Umsetzung einer aromatischen Hydroxyverbindung mit Kohlenmonoxid und Sauerstoff in Gegenwart von Katalysatoren enthaltend
(i) Siliziumdioxid
(ii) ein oder mehrere cokatalytisch wirkende Metalloxide des Mangans und/oder des Cers und
(iii) Palladium oder eine oder mehrere Verbindungen des Palladiums in einer Menge von 0,01 bis 15 Gew.-%, gerechnet als metallisches Palladium und bezogen auf das Gesamtgewicht des Katalysators,
die erhalten werden durch Herstellung eines Gels aus einem oder mehreren geeigneten Vorläufer(n) jeder der unter (i) und (ii) genannten Komponenten sowie der Palladium komponente (iii), Altern, Trocknen und gegebenenfalls Tempern des Gels, eines quaternären Ammonium- oder Phosphonium-Salzes und einer Base.

Das erfindungsgemäße Gel kann nach nahezu allen bekannten Methoden hergestellt werden. Bevorzugt werden Methoden verwendet, die für die Herstellung von Mischoxiden auf Gelbasis bekannt sind. Dies umfaßt beispielsweise die Hydrolyse von einem oder mehreren Metallalkoxiden und/oder hydrolisierbaren Metallverbindungen unter sauren, neutralen oder basischen Bedingungen in geeigneten Lösungsmitteln bei Temperaturen von 0°C bis 200°C. Dabei können auch Mischungen verschiedener Vorläufer eines oder mehrerer Elemente verwendet werden.

Geeignete Vorläufer für Siliziumdioxid sind Alkoxide des Siliziums wie beispielsweise Tetraethoxysilan, Tetramethoxysilan.

Geeignete Lösungsmittel sind beispielsweise einwertige Alkohole wie Methanol, Ethanol, n-Propanol, Isopropanol, n-Butanol, 2-Butanol, tert-Butanol, mehrwertige Alkohole wie Glykol, 1,2-Propandiol, 1,3-Propandiol, ein oder mehrwertige Ketone, wie Aceton, 1,3-Pentandion (Acetylaceton), cyclische oder lineare Ether mit 1 bis 3 Sauerstoffatomen wie Tetrahydrofuran, Dioxan, Diethylether, Glykoldiethylether oder Diethylenglykoldiethylether, Etheralkohole wie Glykolmonomethylether, Nitrile wie Acetonitril und Benzonitril und Amide wie Dimethylformamid. Bevorzugt sind Alkohole, Diketone und Etheralkohole. Selbstverständlich können auch Mischungen der Lösungsmittel eingesetzt werden.

Die Lösungsmittel werden in solchen Mengen eingesetzt, daß das Molverhältnis von Alkoxid zu Lösungsmittel 1:0,2 bis 1:100 beträgt.

Im erfindungsgemäßen Prozess können auch teilweise alkylierte Vorläufer R¹ ₓSi(OR²)_{y} eingesetzt werden, (x+y) die Wertigkeit der Elemente, und R¹ und R² unabhängig voneinander Alkyl-Aralkyl oder Arylreste mit 1-20 Kohlenstoffatomen sind. Beispielsweise seien genannt: Methyltriethoxysilan, Ethyltriethoxysilan.

Als cokatalytisch wirkende Komponenten werden eine oder mehrere Verbindungen des Mangans und/oder des Cers, mit einem molaren Gesamtanteil der unter (ii) genannten Komponenten von 0,1% bis 99,9%, bevorzugt 0,1% bis 40%, besonders bevorzugt 0,5% bis 20%, bezogen auf die Gesamtmolmenge der unter (i) und (ii) genannten Komponenten, in den Katalysator eingebracht.

Geeignete Vorstufen für die cokatalytisch wirksamen Metalle sind grundsätzlich bekannt, beispielsweise können anorganische Salze wie Halogenide, Oxide, Nitrate, Sulfate, Carboxylate, Salze ein oder mehrfunktioneller organischer C₂ bis C₁₅-Carbonsäuren wie Acetate, Cyclohexanbutyrate, Diketonate wie Acetylacetonate, Ethylhexanoate, Alkoxide wie Methoxide, Ethoxide, Isopropoxide, sowie Komplexverbindungen, die beispielsweise Kohlenmonoxid, Olefine, Amine, Nitrile, Phosphine und Halogenide enthalten sowie gemischte Salze eingesetzt werden.

Auch geeignete heterometallische Alkoxide der Formel [LₘM-(OR)₂-M'Lₙ'] sind bekannt und z.B. von Mehrotra et al. in Mat. Res. Soc. Symp.Proc. 121 (1988) 81; D.C. Bradley et al.: "Metal Alkoxides", Academic Press, NY (1978); K.G. Caulton et al. in Chem. Rev. 90 (1990) 969 beschrieben.

Als Beipiele für Verbindungen mit organischen Liganden seien genannt: Cer(IV)isopropoxid, Cer(IV)methoxyethoxid, Cer(III)acetylacetonat, Mangan(II)ethoxid, Mangan(II)-acetylacetonat, Mangan(III)acetylacetonat.

Geeignete Pd-Verbindungen sind beispielsweise die in EP-A 736 324 beschriebenen Palladium verbindungen und Platinmetall-enthaltenden Komplexverbindungen. Weitere Beispiele geeigneter Pd- Verbindungen sind: Li₂(PdCl₄), Na₂(PdCl₄), K₂(PdCl₄), (NBu₄)₂(PdCl₄), Na₂(PdBr₄), K₂(PdBr₄), (NBu₄)₂(PdBr₄) mit (Bu = n-Butyl). Beispiele für olefinhaltige Palladium komplexe sind [Allylpalladiumchlorid]-Dimer - [C₃H₅PdCl]₂, 1,5-Cyclo-octadienpalladiumdichlorid - C₈H₅PdCl₂, Beispiele für phosphinhaltige Pd- komplexe sind 1,2-Bis(diphenylphosphino)ethan]palladiumdichlorid - Pd[(C₆H₅)₂PCH₂CH₂P(C₆H₅)₂]Cl₂, Bis triphenylphosphino)palladiumdichlorid-Pd[P(C₆H₅)₃]₂Cl₂, Beispiele für aminhaltige Palladiumkomplexe sind Diaminopalla- diumdibromid -Pd(NH₃)₂Br₂, Diamminopalladiumdichlorid -Pd(NH₃)₂Cl₂, Tetraamminopalladiumtetrachloropalladat - [Pd(NH₃)₄][PdCl₄], Beispiele für nitrilhaltige Pd-komplexe sind Bis(acetonitril)palladiumdichlorid - Pd(CH₃CN)₂Cl₂, Bis(benzonitril)palladiumdichlorid, Pd(C₆H₅CN)₂Cl₂, Beispiele für kohlenmonoxidhaltige Palladium komplexe sind Tetrabutylammoniumtribromocarbonylpalladat - (NBu₄)Pd(CO)Br₃ (mit Bu=n-Butyl) und Tetrabutylammoniumtrichlorocarbonylpalladat - (NBu₄)Pd(CO)Cl₃ (mit Bu=n-Butyl).

Die Herstellung der erfindungsgemäßen Katalysatoren kann in einem oder mehreren Schritten erfolgen. Dabei wird das Palladium bei der Herstellung des Mischoxids sofort oder zeitlich verzögert in die Mischung eingebracht. Auch Vorgehensweisen, nach denen ein Teil der Platinmetallmenge in den Sol-Gel-Prozeß eingebracht wird, und der Rest später auf das Mischoxid aufgebracht wird, sind möglich.

Bei der Herstellung der erfindungsgemäßen Katalysatoren wird üblicherweise eine Lösung aus den Vorstufen für (i) und für (ii) in einem geeigneten Lösungsmittel hergestellt und mit 1 bis 20, bevorzugt 1,5 bis 10 Moläquivalenten Wasser bezogen auf die Gesamtmolmenge der Verbindungen (i) und (ii) hydrolysiert. Das Wasser kann auf einmal oder in mehreren Portionen, rein, als Mischung mit anderen Lösungsmitteln oder zusammen mit darin gelösten Vorläufern für (ii) oder gelösten Palladium verbindungen zugegeben werden.

Erfindungsgemäß können zu verschiedenen Zeitpunkten eine oder mehrere Verbindungen des Palladiums in einer Menge von 0,01 - 20 Gew.-%, bevorzugt 0,05 - 10 Gew.-%, gerechnet als Pd und bezogen auf das Gesamtgewicht des fertigen Katalysators, in den Katalysator eingebracht werden.

In einer bevorzugten Variante der Katalysatorherstellung werden ein(oder mehrere Palladium verbindungen vor, während oder nach der Gelierung zur oben beschriebenen Mischung der Vorläufer von (i), (ii), dem Lösungsmittel, dem Wasser und ggf. den Säuren oder Basen als Lösung in einem geeigneten Lösungsmittel zugesetzt.

Es kann auch aus einer unlöslichen Vorstufe der Palladium verbindung mit Hilfe eines Komplexbildners oder eines weiteren Liganden eine lösliche Verbindung in situ gebildet werden. In einer anderen bevorzugten Ausführungsform wird die Palladium- verbindung in dem verwendeten Lösungsmittel gelöst und so zugesetzt. In einer weiteren bevorzugten Ausführungsform wird die Palladium verbindung als Feststoff oder Lösung zur bereits vorhydrolysierten Mischung gegeben.

Bei der Hydrolyse können Säuren oder Basen in Mengen von 0,1 bis 200 Mol% bezogen auf die Gesamtmolmenge der Verbindungen (i) und (ii) zugegen sein.

Geeignete Säuren sind beispielsweise Salzsäure, Salpetersäure, Schwefelsäure, Ameisensäure, Essigsäure oder höhere Carbonsäuren mit bis zu 3-8 Kohlenstoffatomen. Ebenfalls geeignet sind Di- und Tricarbonsäuren mit bis zu 8 Kohlenstoffatomen. Geeignete Basen sind Ammoniak, quaternäre Ammoniumhydroxide NR₄OH, wobei die Reste R unabhängig voneinander Alkyl, Aryl oder Aralkylreste mit 1-15 Kohlenstoffatomen sein können, z.B. Tetramethyl-, Tetraethyl, Tetrapropyl, Tetrabutyl-, Tetrapentyl- oder Tetraphenylammoniumhydroxid, oder organische Stickstoffbasen wie Amine, Pyridine, Guanidine. Bevorzugte Basen sind Ammoniak und quaternäre Ammoniumhydroxide. Die Säuren und Basen können als Reinsubstanzen, als wasserfreie Lösungen oder als wäßrige Lösungen eingesetzt werden.

Bei der Zugabe der einzelnen Komponenten ist eine gute Homogenisierung der Mischung durch entsprechende Mischvorrichtungen wie z.B. Rührer oder Mischdüsen sicherzustellen

Werden mehrere Verbindungen (i) und (ii) hydrolysiert, können bekannte Techniken, um ihre Reaktivität aufeinander abzustimmen verwandt werden, beispielsweise seien genannt Prähydrolyse einer Verbindung, chemische Modifikation einer Verbindung mit einem Chelatbildner, Verwenden verschiedener Alkoxidreste in den Verbindungen sowie Hydrolyse bei verschiedenen Temperaturen, wie beispielsweise von D. A. Ward und E.I. Ko (Ind. Eng. Chem. Res. 34 (1995) 421) beschrieben.

Eine weitere geeignete Methode zur Herstellung erfindungsgemäßer Mischungen aus Vorläufern für (i) und (ii) ist die Gelierung anorganischer Vorläufer in wäßrigen Systemen, wie die Herstellung von Kieselgelen durch Neutralisation von Alkalimetallsilikaten mit starken Säuren genannt. Gegebenenfalls sind zusätzliche Schritte wie das Waschen des Gels erforderlich, um gebildete Salze aus der Mischung herauszuwaschen. Bei der hier beschriebenen Vorgehensweise können die Vorläufer für (ii) oder die Platinmetall enthaltenden Verbindungen (iii) beispielsweise vor dem Vermischen von Alkalimetallsilikat und Säure einer der Komponenten zugesetzt werden.

Nach der Gelierung ist es vorteilhaft, die Gele bei Temperaturen von 20-100°C, bevorzugt 20-80°C für eine Zeit von mindestens 10 Minuten altern zu lassen. Die Obergrenze der Alterungszeit ist nur durch ökonomische Faktoren begrenzt und kann mehrere Wochen betragen. Bevorzugt sind Zeiten zwischen einer Stunde und zwei Wochen. Die Alterung kann auch in mehreren Schritten bei verschiedenen Temperaturen oder bei zeitlich langsam veränderter Temperatur erfolgen.

Anschließend an die Alterung werden die Gele getrocknet. Die Trocknung der Gele kann in Abhängigkeit von ihrer Präparation nach verschiedenen Methoden erfolgen, wobei durch die Trocknung die innere Oberfläche und das Porenvolumen der Materialien beinflußt werden.

Die Trocknung kann zum einen an der Luft, im Vakuum oder im Gasstrom geschehen. Geeignete Gase für die Trocknung des Gels im Gasstrom sind Stickstoff, Sauerstoff, Kohlendioxid oder Edelgase sowie beliebige Mischungen der genannten Gase, bevorzugt z.B. Luft. Ebenfalls eignen sich gasförmige Kohlenwasserstoffe zum Beispiel Alkane wie Methan, Ethan, Propan, Butan, Alkene wie Ethen, Propen, Butene, Butadien und Alkine wie Ethin, Propin usw. in beliebiger Zusammensetzung. Die Trocknung erfolgt bei 0 bis 300°C, bevorzugt bei 20 bis 250°C, besonders bevorzugt bei 20 bis 150°C. Die Trockenzeit hängt z.B. von der Porosität des Gels und vom verwendeten Lösungsmittel ab. Sie beträgt im Allgemeinen einige Stunden, beispielsweise 0,5 bis 50 h, bevorzugt 1 bis 40 h, besonders bevorzugt 1 bis 30 h.

Eine andere bevorzugte Methode ist die Trocknung unter überkritischen Bedingungen, wie sie beispielsweise von G.M Pajonk (Applied Catalysis 72 (1991) 217) und Dutoit et al. (J. Catal. 161 (1996) 651) beschrieben werden, und die zu Gelen mit besonders hoher Porosität führt. Beispielsweise kann mit Kohlendioxid (T_{kritisch} = 31°C, p_{kritisch} = 73 bar) oder mit Alkoholen oberhalb des kritischen Punktes (z.B. für Ethanol T_{kritisch} = 243°C, p_{kritisch} = 63 bar) gearbeitet werden. Die Trocknung kann absatzweise, kontinuierlich oder teilkontinuierlich, gegebenenfalls in Anwesenheit eines weiteren Inertgases durchgeführt werden.

Bei der überkritischen Trocknung mit Alkoholen kann gegebenenfalls eine Reduktion des Palladiums auftreten, die sich im allgemeinen negativ auf die Aktivität der erfindungsgemäßen Katalysatoren auswirkt. In diesen Fällen empfiehlt es sich, die Katalysatoren nach der Trocknung wieder zu oxidieren, beispielsweise durch eine Temperung bei 200-800°C in einem Gasstrom; der Sauerstoff, Luft, Halogene oder Halogenwasserstoffe enthält.

Weitere Methoden der Trocknung besonders für in wäßrigen Systemen hergestellte Gele sind extraktive und azeotrope Trocknung, wie sie beispielsweise in US-A 3 887 494, US-A 3 900 457, US-A 4 169 926, US-A 4 152 503, US-A 4 436 883, US-A 4 081 407 beschrieben werden.

Nach der Trocknung können die getrockneten Mischoxide kalziniert werden. Die Kalzinierung kann an der Luft, im Vakuum oder im Gasstrom geschehen. Geeignete Gase für die Kalzinierung der Mischoxide im Gasstrom sind z.B. Stickstoff Sauerstoff, Kohlendioxid oder Edelgase sowie beliebige Mischungen der genannten Gase, bevorzugt Luft. Die Kalzinierung erfolgt bei 100 bis 800°C, bevorzugt bei 100 bis 700°C, besonders bevorzugt bei 100 bis 600°C. Hierbei kann es gegebenenfalls von Vorteil sein, wenn während der Kalzinierung die Zusammensetzung des Gases sprunghaft oder kontinuierlich geändert wird. Die Kalzinierungszeit beträgt im allgemeinen einige Stunden, beispielsweise 0,5 bis 50 h, bevorzugt 1 bis 40 h, besonders bevorzugt 1 bis 30 h.

Es ist ebenfalls möglich, die erfindungsgemäßen Mischoxide als Schicht auf andere Katalysatorträger aufzubringen. Als Trägermaterialien für das Aufbringen einer Schicht aus Metallmischoxid eignen sich alle technisch üblichen Katalysatorträger auf der Basis von Kohle, Elementoxiden, Elementcarbiden oder Elementsalzen in verschiedenen Anwendungsformen. Beispiele für kohlenstoffhaltige Träger sind Koks, Graphit, Ruß oder Aktivkohle. Beispiele für Elementoxid-Katalysatorträger sind SiO₂ (natürliche oder synthetische Kieselsäuren, Quarz), Al₂O₃ in verschiedenen Modifikationen (α, γ, δ, η, θ), Tonerden, natürliche und synthetische Alumosilicate (Zeolithe), TiO₂ (Rutil, Anatas), ZrO₂ oder ZnO. Beispiele für Elementcarbide und -salze sind SiC, AlPO₄, BaSO₄, CaCO₃ u.a. Sie können sowohl im Sinne chemisch einheitlicher Reinsubstanzen als auch im Gemisch eingesetzt werden. Für die erfindungsgemäße Verwendung eignen sich sowohl pulverförmige als auch stückige Materialien, auch Monolithe.

Die Erfindung betrifft ein Verfahren zur Herstellung eines organischen Carbonats durch Umsetzung einer aromatischen Hydroxyverbindung mit Kohlenmonoxid und Sauerstoff in Gegenwart der Katalysatoren, eines quaternären Ammonium- oder Phosphonium-Salzes und einer Base.

Das mit dem erfindungsgemäßen Verfahren hergestellte organische Carbonat entspricht der Formel

R-O-CO-O-R (I),

in der
- R: ein substituiertes oder nicht substituiertes C₆-C₁₂-Aryl, bevorzugt ein substituiertes oder nicht substituiertes Phenyl, besonders bevorzugt nicht substituiertes Phenyl bedeutet.

Die erfindungsgemäß einsetzbaren aromatischen Hydroxyverbindungen entsprechen der Formel

R-O-H (II),

in der R die oben angegebene Bedeutung hat. Bei den mit den erfindungsgemäßen Träger-Katalysatoren umsetzbaren aromatischen Hydroxyverbindungen handelt es sich beispielsweise um Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenof, 1-Naphthol, 2-Naphthol oder Bisphenol A, bevorzugt um Phenol. Die aromatische Hydroxyverbindung kann mit 1 oder 2 Substituenten in der Bedeutung von C1-C₄-Alkyl, C₁-C₄-Alkoxy, Fluor, Chlor oder Brom substituiert sein.

Die Katalysatoren können als Pulver, Formkörper oder Monolithe eingesetzt werden, bevorzugt als Pulver oder Formkörper, und aus der Reaktionsmischung z.B. durch Filtration, Sedimentation oder Zentrifugieren abgetrennt werden.

Die Herstellung von aromatischen Carbonaten mit den Träger-Katalysatoren kann in verschiedenen Ausführungsvarianten ausgeübt werden. Eine Möglichkeit besteht in der diskontinuierlichen Durchführung. Im Falle einer kontinuierlichen Fahrweise im Gegen- oder Gleichstrom oder in der Rieselphase am Festbett-Katalysator werden Belastungen von 0,01 bis 20 g aromatische Hydroxyverbindung pro Gramm Träger-Katalysator und Stunde, bevorzugt 0,05 bis 10 g aromatische Hydroxyverbindung pro Gramm Träger-Katalysator und Stunde, besonders bevorzugt 0,1 bis 5 g aromatische Hydroxyverbindung pro Gramm Träger-Katalysator und Stunde eingestellt. Die in diskontinuierlichen Versuchen verwendeten Träger-Katalysatoren können bei gleichen Einsatzstoffen ohne Reinigung wiederholt eingesetzt werden. Bei kontinuierlicher Arbeitsweise können die eingesetzten Träger-Katalysatoren über lange Zeit im Reaktor verbleiben. Bevorzugt kommt bei der Verwendung Träger-Katalysatoren eine kontinuierliche Arbeitsweise im Einzelreaktor oder in einer Kaskade aus Reaktoren zum Einsatz.

Wird der Träger-Katalysator als Pulver eingesetzt, sind zur Vermischung der Reaktionskomponenten die zu verwendenden Rührbehälter mit dafür brauchbaren Rührern ausgestattet. Beim Arbeiten mit Träger-Katalysator-Pulvern als Suspension in Rührgefäßen oder Blasensäulen werden Mengen von 0,001 bis 50 Gew.-%, bevorzugt von 0,01 bis 20 Gew.-%, besonders bevorzugt von 0,1 bis 10 Gew.-% Träger-Katalysator-Pulver, bezogen auf die eingesetzte Menge an aromatischer Hydroxyverbindung, verwendet. In besonders bevorzugten Ausführungsformen wird der heterogene Träger-Katalysator als Formkörper ortsfest in Rührkesseln, Blasensäulen, Rieselphasenreaktoren oder Kaskaden dieser Reaktoren, wobei die verschiedenen Reaktortypen auch gleichzeitig in einer Kaskade vorkommen können, eingesetzt.

Für den Fall der Anordnung des Katalysators als Festbett wird der Katalysator vorzugsweise als Formkörper, z.B. als Kugeln, Zylinder, Stäbchen, Hohlzylinder, Ringe, usw. eingesetzt. Wahlweise können Katalysatoren weiter durch Extrudieren, Tablettieren, gegebenenfalls unter Zumischen weiterer Katalysatorträger oder Bindemittel wie SiO₂ oder Al₂O₃, und Kalzinieren modifiziert werden. Darstellung und Weiterverarbeitung der erfindungsgemäß verwendeten Katalysatoren sind dem Fachmann wohl bekannt und Stand der Technik.

Für das erfindungsgemäße Verfahren können beliebige sowohl organische als auch anorganische Basen oder Mischungen derselben verwendet werden. Als Beispiele für anorganische Basen seien, ohne das erfindungsgemäße Verfahren einzuschränken, Alkalimetallhydroxide und -carbonate, -carboxylate oder andere Salze schwacher Säuren sowie Alkalisalze von aromatischen Hydroxyverbindungen der Formel(II), z.B. Alkalimetallphenolate, genannt. Selbstverständlich können in das erfindungsgemäße Verfahren auch die Hydrate von Alkalimetallphenolaten eingesetzt werden. Als Beispiel für ein solches Hydrat sei hier, ohne das erfindungsgemäße Verfahren einzuschränken, Natriumphenolat-trihydrat genannt. Die Menge an zugesetztem Wasser ist jedoch vorzugsweise so bemessen, daß pro Mol Base maximal 5 Mol Wasser eingesetzt werden. Höhere Wasserkonzentrationen führen i.a. zu schlechteren Umsätzen und Zersetzung gebildeter Carbonate. Als organische Basen seien, ohne das erfindungsgemäße Verfahren einzuschränken, tertiäre Amine, die als organische Reste C₆bis C₁₀-Aryl-, C₇- bis C₁₂-Aralkyl- und/oder C₁- bis C₂₀-Alkyl-Reste tragen können oder Pyridinbasen oder hydrierte Pyridinbasen darstellen genannt, beispielsweise Triethylamin, Tripropylamin, Tributylamin, Trioctylamin, Benzyldimethylamin, Dioctylbenzylainin, Dimethylphenethylamin, 1-Dimethylarrvno-2-phenylpropan, Pyridin, N-Methylpiperidin, 1,2,2,6,6-Pentamethylpiperidin. Bevorzugt wird als Base ein Alkalisalz einer aromatischen Hydroxyverbindung verwendet, besonders bevorzugt ein Alkalisalz der aromatischen Hydroxyverbindung, die auch zum organischen Carbonat umgesetzt werden soll. Diese Alkalisalze können Lithium-, Natrium-, Kalium-, Rubidium- oder Cäsiumsalze sein. Bevorzugt werden Lithium-, Natrium-, und Kaliumphenolat, besonders bevorzugt Natriumphenolat eingesetzt.

Die Base kann dem Reaktionsgemisch als reine Verbindung in fester Form oder als Schmelze zugesetzt werden. In einer weiteren Ausführungsform der Erfindung wird die Base dem Reaktionsgemisch als Lösung, die 0,1 bis 80 Gew.-%, bevorzugt 0,5 bis 65 Gew.-%, besonders bevorzugt 1 bis 50 Gew.-% der Base enthält, zugesetzt. Als Lösungsmittel können hierbei sowohl Alkohole oder Phenole, wie z.B. das umzusetzende Phenol, als auch inerte Lösungsmittel verwendet werden. Als solche seien die weiter unten als Reaktionmedien erwähnten genannt. Diese Lösungsmittel können allein oder in beliebiger Kombination miteinander eingesetzt werden. So besteht eine Ausführungsform des erfindungsgemäßen Verfahrens beispielsweise darin, daß man die Base in einer Phenolschmelze löst, die mit einem Lösungsmittel verdünnt wurde. Bevorzugt wird die Base in der Schmelze einer aromatischen Hydroxyverbindung gelöst, besonders bevorzugt in einer Schmelze der aromatischen Hydroxyverbindung, die zum organischen Carbonat umgesetzt werden soll. Ganz besonders bevorzugt wird die Base, in Phenol gelöst, zugesetzt. Die Base wird in einer von der Stöchiometrie unabhängigen Menge zugesetzt. Das Verhältnis von Platinmetall, z.B. Palladium zu Base wird vorzugsweise so gewählt, daß pro Mol Platinmetall, z.B. Palladium 0,1 bis 500, bevorzugt 0,3 bis 200 besonders bevorzugt 0,9 bis 130 Äquivalente Base bezogen auf Platinmetall, z.B. Palladium eingesetzt werden.

Das erfindungsgemäße Verfahren wird vorzugsweise ohne Lösungsmittel durchgeführt. Selbstverständlich können auch inerte Lösungsmittel verwendet werden. Als Beispiele für Lösungsmittel seien Dimethylacetamid, N-Methylpyrrolidinon, Dioxan, t-Butanol, Cumylalkohol, Isoamylalkohol, Tetramethylharnstoff, Diethylenglykol, halogenierte Kohlenwasserstoffe (z.B. Chlorbenzol oder Dichlorbenzol) und Ether genannt.

Bei den im Rahmen der vorliegenden Erfindung eingesetzten quaternären Salzen kann es sich beispielsweise um mit organischen Resten substituierte Ammonium- oder Phosphoniumsalze handeln. Geeignet für den Einsatz im erfindungsgemäßen Verfahren sind Ammonium- und Phosphoniumsalze, die als organische Reste C₆- bis C₁₀-Aryl-, C₇- bis C₁₂-Aralkyl- und/oder C₁- bis C₂₀-Alkyl-Reste und als Anion ein Halogenid, Tetrafluoroborat oder Hexafluorophosphat tragen. Bevorzugt werden in das erfindungsgemäße Verfahren Ammoniumsalze, die als organische Reste C₆- bis C₁₀-Aryl-, C₇- bis C₁₂-Aralkyl- und/oder C₁- bis C₂₀-Alkyl-Reste und als Anion ein Halogenid tragen, eingesetzt, besonders bevorzugt ist Tetrabutylammoniumbromid. Die Menge eines solchen quaternären Salzes beträgt 0,1 bis 50 Gew.-%, bezogen auf das Gewicht des Reaktionsgemisches. Vorzugsweise beträgt diese Menge 0,5 bis 15 Gew.-%, besonders bevorzugt 1 bis 5 Gew.-%.

Das erfindungsgemäße Verfahren wird, vorzugsweise ohne Lösungsmittel, bei 30 bis 200°C, bevorzugt bei 30 bis 150°C, besonders bevorzugt bei 40 bis 120°C bei einem Druck von 1 bis 100 bar, bevorzugt von 2 bis 50 bar, besonders bevorzugt bei 5 bis 25 bar durchgeführt.

### Beispiele

### Vergleichsbeispiel 1 (nach EP-A 736 324)

### Herstellung eines pulverförmigen Manganoxid-Trägers:

Zu einer Lösung von 126 g Mangan(II)-chlorid (1 mol) in 500 ml Wasser wurden 85 g Natriumhydroxid (2,125 mol) gelöst in 200 ml Wasser zugetropft. Der so erhaltene Niederschlag wurde abgesaugt, gewaschen und getrocknet. Anschließend wurde er 3h bei 300°C und 2h bei 500°C getempert.

### Belegung des pulverförmigen Manganoxids mit Palladium:

Zu einer Aufschlämmung von 292,5 g Mangandioxid-Pulver in 1500 ml Wasser wurden bei Raumtemperatur 300 ml Lösung von 50 g Natriumtetrachloropalladat(II)-Lsg. mit 15% Palladium in Wasser gegeben. Anschließend wurde mit verdünnter Natronlauge alkalisch gestellt. Die Suspension wurde abgesaugt und bei 100°C getrocknet. Der Kontakt enthält 2,5% Pd auf MnO₂-Träger, gerechnet als Metall.

### Einsatz des Träger-Katalysators zur Herstellung von Diphenylcarbonat:

In einem Autoklaven (1) mit Begasungsrührer, Kühler und nachgeschalteter Kühlfalle wurden bei 70°C 8,31 g Tetrabutylammoniumbromid und 0,77 g Mangan(II)acetylacetonat in 450 g Phenol gelöst. Dann wurden 4 g des oben beschriebenen Träger-Katalysators und 2,21 g Natriumphenolat, gelöst in 50 g Phenol, zugegeben. Unter Einleitung eines Gasgemisch aus Kohlenmonoxid und Sauerstoff (95:5 Vol.-%) wurde dann der Druck auf 14 bar eingestellt. Die Menge an Gasgemisch wurde auf 350 Nl/h eingestellt. Dem Reaktionsgemisch wurde jede Stunde eine Probe entnommen und gaschromatographisch analysiert. Die Analysen ergaben, daß nach einer Stunde 9,9 % Diphenylcarbonat, nach 2 Stunden 15,2 % Diphenylcarbonat und nach 3 Stunden 18,2 % Diphenylcarbonat im Reaktionsgemisch enthalten waren. In der Kühlfalle waren 11,8 g eines Phenol/Wasser-Gemisches kondensiert.

### Vergleichsbeispiel 2 (nach EP-A 736 325)

### Belegung eines pulverförmigen Titandioxids mit Palladium und Mangan:

Zu einer Aufschlämmung von 283,5 g Titandioxid-Pulver (Norton) in 1500 ml Wasser wurden bei Raumtemperatur 300 ml Lösung von 40,5 g (0,16 mol) Mangan(II)-nitrat-4-hydrat in Wasser gegeben. Dann wurde mit verdünnter Natronlauge alkalisch gestellt. Die Suspension wurde abgesaugt, mit Wasser gewaschen, bei 100°C getrocknet und 3h bei 300°C getempert. Der mit Mangan dotierte Träger wurde in 1500 ml Wasser aufgeschlämmt und mit 300 ml Lösung, enthaltend 50 g Natriumtetrachloropalladat(II)-Lsg. mit 15% Palladium, versetzt. Dann wurde mit verdünnter Natronlauge alkalisch gestellt. Die Suspension wurde abgesaugt, gewaschen und bei 100°C getrocknet.

Der Katalysator enthält 2,5 % Pd und 3 % Mn, jeweils als Metall gerechnet.

### Einsatz des Träger-Katalysators zur Herstellung von Diphenylcarbonat:

Der Einsatz des Träger-Katalysators zur Herstellung von Diphenylcarbonat erfolgte wie in Vergleichsbeispiel 1. Die Analysen ergaben, daß nach einer Stunde 9,6 % Diphenylcarbonat, nach 2 Stunden 16,1 % Diphenylcarbonat und nach 3 Stunden 21,0 % Diphenylcarbonat im Reaktionsgemisch enthalten waren. In der Kühlfalle waren 12,3 g eines Phenol/Wasser-Gemisches kondensiert.

### Beispiel 1

### Herstellung eines Si/Mn/Pd-Cogels

100 mL Tetraethoxysilan wurden mit einer Lösung von 6,9 g Mangan(III)acetylacetonat und 1,24 g Palladium(II)acetylacetonat in 200 mL Ethanol vermischt und innerhalb von 18 Minuten unter Rühren 36 mL 25,7%ige wäßrige Salzsäure zugegeben. Die Mischung wurde 6 Tage bei Raumtemperatur stehengelassen und anschließend 2 Tage bei 40°C im Vakuumtrockenschrank getrocknet. Der so erhaltene Feststoff wurde vermahlen und 3 Stunden bei 300°C im Luftstrom getempert.

Der Katalysator enthält 1,5 % Palladium und 3,0 % Mangan jeweils als Metall gerechnet.

### Einsatz des Träger-Katalysators zur Herstellung von Diphenylcarbonat:

Der Einsatz des Träger-Katalysators zur Herstellung von Diphenylcarbonat erfolgte wie in Vergleichsbeispiel I, jedoch mit dem Unterschied, daß 6,7 g Katalysator eingesetzt wurden. Die Analysen ergaben, daß nach einer Stunde 9,2 % Diphenylcarbonat, nach 2 Stunden 17,8 % Diphenylcarbonat und nach 3 Stunden 24,4 % Diphenylcarbonat im Reaktionsgemisch enthalten waren. In der Kühlfalle waren 15,0 g eines Phenol/Wasser-Gemisches kondensiert.

### Beispiel 2

### Herstellung eines Si/Mn/Pd-Cogels

Der Katalysator wurde hergestellt, wie in Beispiel 1 beschrieben, mit der Abweichung, daß 13,8 g Mangan(III)acetylacetonat, 2,48 g Palladium(II)acetylacetonat und 300 mL Ethanol verwendet wurden.

Der Katalysator enthält 3,0 % Palladium und 6,0 % Mangan jeweils als Metall gerechnet.

### Einsatz des Träger-Katalysators zur Herstellung von Diphenylcarbonat:

Der Einsatz des Träger-Katalysators zur Herstellung von Diphenylcarbonat erfolgte wie in Beispiel 1, jedoch mit dem Unterschied, daß 3,3 g Katalysator eingesetzt wurden. Die Analysen ergaben, daß nach einer Stunde 11,6 % Diphenylcarbonat, nach 2 Stunden 21,4 % Diphenylcarbonat und nach 3 Stunden 27,0 % Diphenylcarbonat im Reaktionsgemisch enthalten waren. In der Kühlfalle waren 16,5 g eines Phenol/Wasser-Gemisches kondensiert.

### Beispiel 3

### Herstellung eines Si/Mn/Pd-Cogels

100 mL Tetraethoxysilan wurden mit einer Lösung von 6,9 g Mangan(III)acetylacetonat in 200 mL Ethanol vermischt und innerhalb von 18 Minuten unter Rühren eine Lösung von 1,24 g Kaliumtetrachloropalladat in 36 mL 25,7%ige wäßrige Salzsäure zugegeben. Die Mischung wurde 3 Tage bei 40°C stehengelassen und anschließend 2 Tage bei 40°C im Vakuumtrockenschrank getrocknet. Der so erhaltene Feststoff wurde vermahlen und 3 Stunden bei 300°C im Luftstrom getempert.

Der Katalysator enthält 1,5 % Palladium und 6,0 % Mangan jeweils als Metall gerechnet.

### Einsatz des Träger-Katalysators zur Herstellung von Diphenylcarbonat:

Der Einsatz des Träger-Katalysators zur Herstellung von Diphenylcarbonat erfolgte wie in Beispiel 1. Die Analysen ergaben, daß nach einer Stunde 11,4 % Diphenylcarbonat, nach 2 Stunden 19,2 % Diphenylcarbonat und nach 3 Stunden 24,2 % Diphenylcarbonat im Reaktionsgemisch enthalten waren. In der Kühlfalle waren 13,9 g eines Phenol/Wasser-Gemisches kondensiert.

### Beispiel 4

### Herstellung eines Si/Mn/Pd-Cogels

Der Katalysator wurde hergestellt wie in Beispiel 1, nur daß anstelle der Salzsäure eine Mischung von 17 ml Eisessig und 30 ml Wasser zur Hydrolyse verwendet wurde.

Der Katalysator enthält 1,5 % Palladium und 6,0 % Mangan jeweils als Metall gerechnet.

### Einsatz des Träger-Katalysators zur Herstellung von Diphenylcarbonat:

Der Einsatz des Träger-Katalysators zur Herstellung von Diphenylcarbonat erfolgte wie in Beispiel 1. Die Analysen ergaben, daß nach einer Stunde 13,4 % Diphenylcarbonat, nach 2 Stunden 19,6 % Diphenylcarbonat und nach 3 Stunden 24,6 % Diphenylcarbonat im Reaktionsgemisch enthalten waren. In der Kühlfalle waren 15,1 g eines Phenol/Wasser-Gemisches kondensiert.

## Patentansprüche

1. Verfahren zur Herstellung eines organischen Carbonats durch Umsetzung einer aromatischen Hydroxyverbindung mit Kohlenmonoxid und Sauerstoff in Gegenwart von Katalysatoren enthaltend
(i) Siliziumdioxid
(ii) ein oder mehrere cokatalytisch wirkende Metalloxide des Mangans und/oder des Cers und
(iii) Palladium oder eine oder mehrere Verbindungen des Palladiums in einer Menge von 0,01 bis 15 Gew.-%, gerechnet als metallisches Palladium und bezogen auf das Gesamtgewicht des Katalysators,
die erhalten werden durch Herstellung eines Gels aus einem oder mehreren geeigneten Vorläufer(n) jeder der unter (i) und (ii) genannten Komponenten sowie der Palladium komponente (iii), Altern, Trocknen und gegebenenfalls Tempern des Gels, eines quaternären Ammonium- oder Phosphonium-Salzes und einer Base.

## Claims

1. A process for preparing an organic carbonate by reacting an aromatic hydroxy compound with carbon monoxide and oxygen in the presence of catalysts containing
(i) silicon dioxide,
(ii) one or more co-catalytic metal oxides of manganese and/or cerium and
(iii)palladium or one or more compounds of palladium in an amount of 0.01 to 15 wt.%, calculated as metallic palladium and with respect to the total weight of catalyst,
which are obtained by preparing a gel from one or more suitable precursor(s) of each of the components mentioned under (i) and (ii) and the palladium component (iii), ageing, drying and optionally annealing the gel, a quaternary ammonium or phosphonium salt and a base.

## Revendications

1. Procédé pour la préparation d'un carbonate organique par réaction d'un composé aromatique hydroxylé avec le monoxyde de carbone et l'oxygène en présence de catalyseurs contenant
(i) de la silice
(ii) un ou plusieurs oxydes métalliques du manganèse et/ou du cérium, faisant fonction de catalyseurs auxiliaires et
(iii) le palladium ou un ou plusieurs composés du palladium en quantité de 0,01 à 15 % en poids, exprimé en palladium métallique et rapporté au poids total du catalyseur,
qui sont obtenus par préparation d'un gel à partir d'un ou plusieurs produits précurseurs appropriés de chacun des composants mentionnés sous (i) et (ii) et du composant palladium (iii), vieillissement, séchage et le cas échéant cuisson du gel, d'un sel d'ammonium ou de phosphonium quaternaire et d'une base.
